(19) Europäisches Patentamt
European Patent Office
Office européen des brevets

(11) Publication number: **0 620 210 A1**

# (12) EUROPEAN PATENT APPLICATION

(21) Application number: **94105463.7**

(22) Date of filing: **08.04.94**

(51) Int. Cl.[5]: **C07C 237/34**, C07D 295/13, A61K 31/165, A61K 31/40, A61K 31/535

(30) Priority: **16.04.93 IT BO930154**

(43) Date of publication of application: **19.10.94 Bulletin 94/42**

(84) Designated Contracting States: **AT BE CH DE DK ES FR GB GR IE LI LU NL PT SE**

(71) Applicant: **ALFA WASSERMANN S.p.A.**
**Contrada Sant'Emidio**
**I-65020 Alanno Scalo (Pescara) (IT)**

(72) Inventor: **Baldazzi, Claudia**
**Via Galvani n. 50**
**I-40064 Ozzano Emilia (Bologna) (IT)**
Inventor: **Piani, Silvano**
**Via Pietro Inviti n.4**
**I-40131 Bologna (IT)**
Inventor: **Barbanti, Maria**
**Mura di Porta d'Azeglio n. 4**
**I-40136 Bologna (IT)**
Inventor: **Marchi, Egidio**
**Via Don Ercolani n. 3**
**I-40033 Casalecchio di Reno (Bologna) (IT)**

(74) Representative: **Kraus, Walter, Dr. et al**
**Patentanwälte Kraus, Weisert & Partner**
**Thomas-Wimmer-Ring 15**
**D-80539 München (DE)**

## (54) Compounds with benzamidic structure active in gastro-intestinal pathologies.

(57) This invention refers to compounds with benzamidic structure of general formula

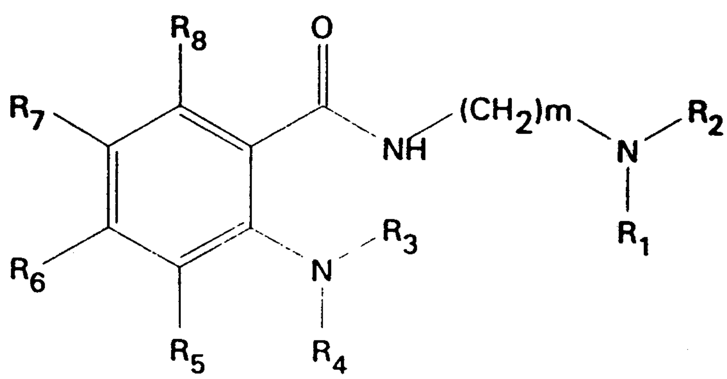

I

and their pharmacologically accepted salts.

The derivatives described in this invention are active in the gastro-intestinal apparatus; in particular, they have prokinetic effects, in other words they create specific stimulation on gastro-intestinal motility, facilitating the passage of the content of the alimentary tract, and possess anti-emetic qualities, without side effects involving the central nervous system.



Rank Xerox (UK) Business Services
(3.10/3.09/3.3.4)

## SCOPE OF THE INVENTION

This invention refers to the synthesis of derivatives with benzamidic structure having favourable effects on gastro-intestinal motility, with prokinetic and anti-emetic properties and without side effects involving the central nervous system. The principal derivatives with prokinetic properties used in therapy belong to the benzamide chemical class and their parent is metoclopramide, of the formula

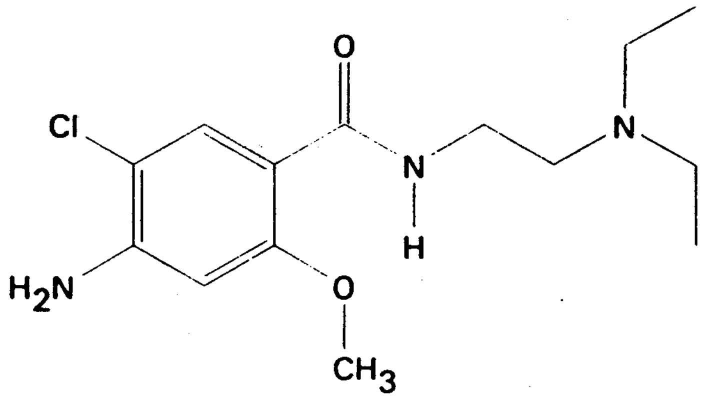

described in the US patent 3,177,252, which stimulates the motility of the upper gastro-intestinal tract with a mechanism which probably implies interaction with receptors $5HT_4$, as reported by Brunnengräber R. et al., Pharma. Zeitung, 35, 9-18, 1991 and by Turconi M. et al., Drugs of the Future, 16, 1011, 1991, and has both central and peripheral antagonistic activity on the dopaminergic receptors $D_2$, as reported by King F.D. and Senger G.J., Ann. Rep. Med. Chem. 23, 201, 1990.

Over the last few years a large number of derivatives of metoclopramide, presenting structural modifications in the lateral chain, have been studied with the aim of leaving the prokinetic activity unaltered, though eliminating antagonism to the dopaminergic receptors. The antagonist action on these receptors, considered secondary in disorders of gastro-intestinal motility, seems to be of particular importance due to a series of undesirable side effects caused by drugs in use, such as extrapyramidal motor symptoms and high levels of prolactin in the blood.

The product currently used in therapy which best fulfils these pharmacological requirements is cisapride, with the formula

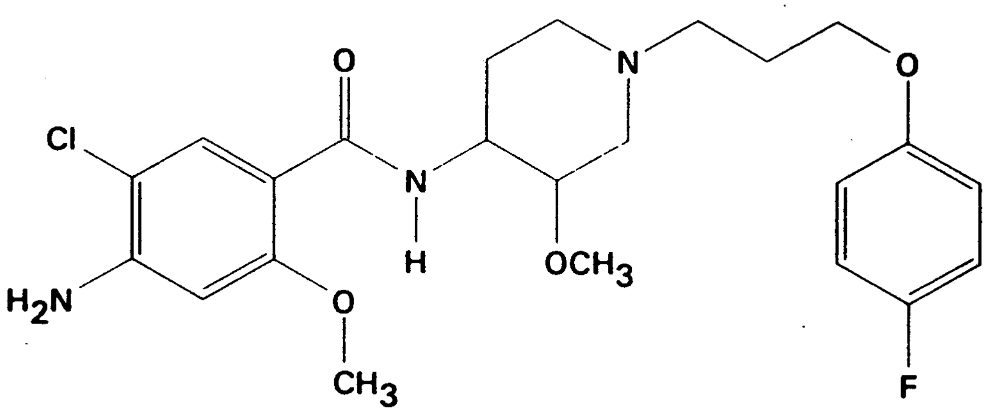

whose synthesis and pharmacological properties are described in the patent EP 0076530 and in Drugs of the Future, 13, 676, 1988.

Craig and Clarke, Brit. J. Pharm., 102, 563-564, 1991, report that cisapride stimulates intestinal motility through interaction with serotoninergic receptors $5HT_4$ and has practically no antagonist property of the dopamine receptors, of cholinesterase inhibition or direct parasympathomimetic activity.

King F.D. and Senger G.J., Ann. Rep. Med. Chem. 23, 202, 1990, report the structures of several other derivatives, structurally modified in lateral chain with respect to metoclopramide, and having prokinetic activity such as BRL 20627, dazopride, clebopride, which, unlike cisapride, show a fair amount of activity on the dopaminergic receptors $D_2$.

It is also known from the state of the art that some derivatives similar to those mentioned have a receptorial selectivity on the receptors $5HT_3$ with effects on the central nervous system such that they may also be used in the therapy of disturbances at a central nervous level.

This invention refers to new derivatives with benzamidic structure, substituted in position 2 of the aromatic ring with a substituted or unsubstituted amino group, obtained by introduction of a chain or group bringing a basic residue on the amidic nitrogen atom, designed according to evaluations of structure-activity on the products already described in the state of the art. The introduced structural variations influence the pharmacological properties of the derivatives, increasing their effect on gastro-intestinal motility and eliminating antagonism with the dopaminergic receptors, with the subsequent elimination of the undesirable effects on the central nervous system and on the endocrine constellation.

The derivatives described in this invention may be gainfully used in the treatment of a number of different pathologies of the gastro-intestinal tract such as nausea and vomit, also subsequent to anti-tumour treatments; gastro-esophageal regurgitation disease; functional dyspepsia from slowed gastric dumping; gastric hypomotility associated with anorexia nervosa; slowed gastric dumping and slowed intestinal transit during treatment with opiate analgesics (or in the case of opium-based drug dependence); gastroparesis of different origins (diabetic neuropathy); delayed gastric dumping; disturbances of gastro-duodenal motor co-ordination and functional disturbances of the upper alimentary tract in general; further applications at pediatric level may be as part of the treatment of disease from gastro-esophageal regurgitation, of pylorospasm or generally in slowed gastric dumping, regurgitations from cardial incontinence and cystic fibrosis.

## DESCRIPTION OF THE INVENTION

This invention refers to new derivatives with benzamidic structure, to their pharmacologically acceptable salts and their therapeutic use in gastro-intestinal pathologies, as stimulators of gastro-intestinal motility, in other words as prokinetics and as antiemetics.

The compounds described in the claims of this invention have the following general formula:

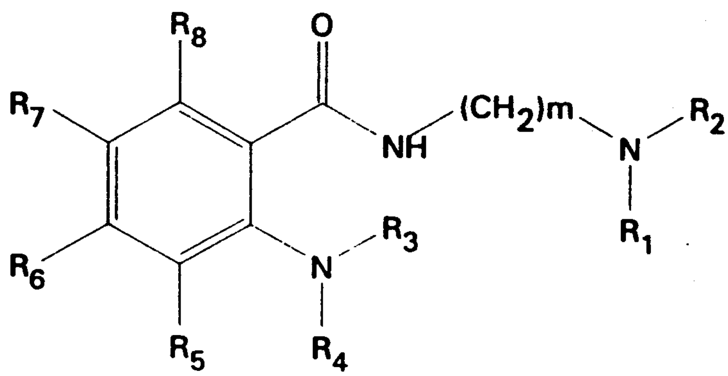

I

where *m* represents a whole number between 1 and 4, $R_1$ and $R_2$, independently, represent an atom of hydrogen or a $(C_1-C_6)$-alkyl radical, with a linear or ramified chain, or $R_1$ and $R_2$ together with the nitrogen atom form a heterocyclic ring. $R_3$ and $R_4$, independently, represent a hydrogen atom or a $(C_1-C_{10})$-alkyl radical, with linear, ramified or cyclic chain, or a benzyl radical.

$R_5$, $R_6$, $R_7$ and $R_8$, independently, represent a hydrogen atom or a $(C_1-C_6)$-alkyl radical, with linear or ramified chain, or an atom of halogen. The compounds preferred in carrying out this invention are those in which $R_1$ and $R_2$, independently, represent a $(C_1-C_3)$-alkyl radical or a hydrogen atom or taken together with the nitrogen atom form a heterocyclic ring, $R_5$, $R_6$ and $R_8$ represent an atom of hydrogen, $R_7$ represents an atom of halogen, preferably an atom of chlorine, and *m* is a whole number chosen between 2 and 3.

The derivatives of general formula I are obtained by means of a process which starts with the synthesis of benzamides of the general formula

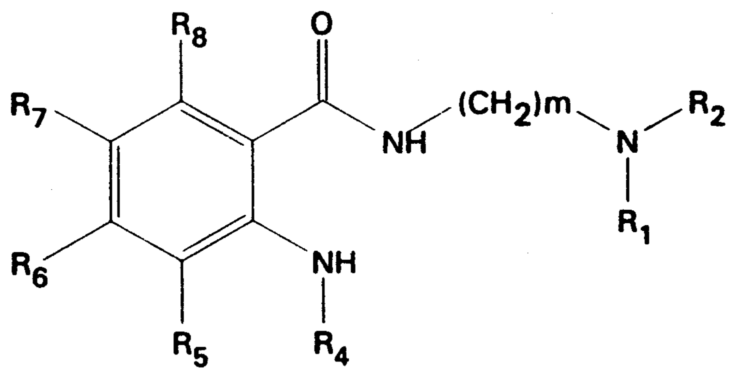

II

in which $R_1$, $R_2$, $R_4$, $R_5$, $R_6$, $R_7$, $R_8$ and *m* have the above-mentioned meanings.

An isatoic anhydride of general formula

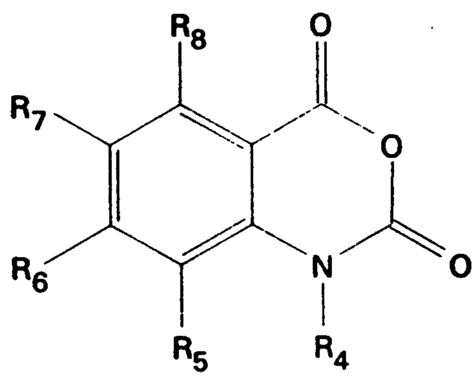

III

is made to react with 1 to 3 molar equivalents of an amine of general formula

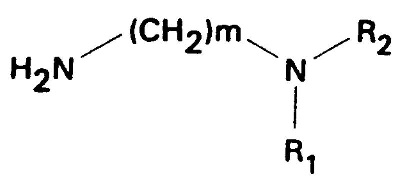

IV

at a temperature of between 15 °C and 70 °C for a period of time between 15 minutes and 24 hours, in an organic solvent selected from linear or cyclic ethers and alcohols containing from 1 to 4 atoms of carbon.

The amides of formula II are isolated through evaporation of the solvent and purified if necessary through chromatography on columns of silica gel.

In the amide of formula II the atom of hydrogen on the amino group in position 2 of the aromatic ring can be substituted by a linear, ramified or cyclic ($C_1$-$C_{10}$)-alkyl radical, or by a benzyl radical, through treatment with an equivalent quantity of a corresponding halide in a polar solvent in the presence of between 1 to 4 molar equivalents of a base, preferably sodium hydride, potassium carbonate or sodium hydroxide, at a temperature between room temperature and the reflux temperature of the reaction mixture for a period of time of between 4 and 48 hours.

The products are recovered by extraction from the reaction mixture with an organic solvent, subsequent evaporation of the solvent and can be further purified by chromatography on silica gel.

The pharmacologically acceptable salts of the compounds of formula I are obtained by cold mixing equimolar quantities or in slight excess (1.1-1.2 eq.) of the suitable acid dissolved in a solvent selected from alcohols containing from 1 to 6 atoms of carbon, cyclic or acyclic ethers or their mixtures with chloroform or methylene chloride, with the compounds of formula I dissolved in mixtures of the same solvents, at a temperature of between 0 °C and room temperature and filtering the precipitated salt. These salts may also be obtained by dissolving in water the acid, in equimolecular quantities or in slight excess (1.1-1.2 eq.), and

the compound of formula I; after evaporation of the solvent the product is crystallized by alcohols containing from 1 to 6 atoms of carbon or by their mixtures.

Salts of inorganic acids such as hydrochloric and sulphuric acids and salts of organic acids, such as oxalic, citric, tartaric, succinic and acetic acids are preferred in carrying out this invention. The salts of citric and oxalic acids are particularly preferred in performing this invention.

The melting point was determined by means of a Büchi melting point instrument, without making any corrections.

The I.R. spectrum was obtained using a Perkin-Elmer spectrophotometer mod. 281/B, usually, when not otherwise specified, preparing the sample in nujol and calibrating the spectrum between 4000 and 600 nm.

The $^{1}$H-NMR spectrum was calibrated at room temperature by means of a Varian Gemini spectrometer at 200 MHz, in the solvents described, using tetramethylsilane as an internal standard; the signal resonances were expressed in p.p.m..

The $^{13}$C-NMR spectrum was performed at 50.3 MHz with a Varian Gemini 200 spectrometer using tetramethylsilane as an internal standard and the solvents described in the experimental section as solvents.

The mass spectrum was calibrated using a mass spectrometer VG 7070E, at 70 eV of ionization voltage and with a voltage of acceleration of 6 Kvolt.

The silica gel chromatographies were done using silica gel 60 F254 (230-400 mesh - Merck) with the eluents described in the examples, according to the method described by Clark Still W. et al. in J. Org. Chem., 43, 2923, 1978.

The biological activity of the new derivatives described in this invention was determined by means of some typical tests, both *in vitro* and *in vivo*, commonly used on compounds featuring the antiemetic and prokinetic activity described.

In tables 5, 6, 7 and 8 the significance was determined by means of Student's test on absolute values with respect to the relative controls using 10 animals per group in tests 5, 6 and 7. The standard errors were found to be less than 10% of the average. The significant values, with significance $\mathbf{p < 0.05}$, have been indicated with an asterisk.

*In vitro* tests were carried out to evaluate the effect of the compounds of formula I and some of their pharmacologically acceptable salts on physiological peristalsis, on the action of the histamine, serotonine and acetylcholine.

The action on physiological peristalsis was evaluated on an isolated rabbit jejunum preparation, according to the method reported by Magnus J. in Pfluegers Arch. Gen. Physiol. 102, 123, 1904 and described by Beretta C. in Metodiche sperimentali di fisiologia e farmacologia, Tamburini Ed., Milano, 1972.

The effect on the histamine was evaluated on a preparation of guinea-pig ileum according to the method described by the above-mentioned authors.

The effect on the serotonine was evaluated on the fornix of the stomach of a rat according to the method of Vane J.R. in Br. J. Pharmacol. Chemother 12, 344, 1957.

Finally the effect on the acetylcholine was evaluated on the ascending colon of a rat, according to the method of Gaddum J. H. and Horton E.W. reported in Br. J. Pharmacol. Chemother. 14, 117, 1959.

For the evaluation of the physiological peristalsis and the action on the histamine, the rabbit jejunum and the guinea-pig ileum were removed from the animals sacrificed after 24 hours fasting, washed with Tyrode fluid at 37°C and perfused with the same oxygenated fluid (95% oxygen and 5% carbon dioxide) at the temperature of 37°C. The rabbit jejunum was used to evaluate the extent of the spontaneous peristaltic contractions in the presence and in the absence of the derivatives being studied.

The effect on the spontaneous peristalsis was found to be analogous to that of cisapride, although with greater concentrations than the reference as shown in Table 1 below. These results in themselves suggest prokinetic activity *in vivo* of these products.

TABLE 1

| Spontaneous peristaltic activity on rabbit jejunum | | | |
|---|---|---|---|
| **example** | **maximum effect %** (max. increase of extent of the contractions) | **concentration required to obtain the maximum effect** | **$CE_{50}$** (concentration able to produce 50% of the maximum effect) |
| **1** | +47.2 | $2 \times 10^{-6}$ M | $2 \times 10^{-8}$ M |
| **2** | +21.1 | $3 \times 10^{-7}$ M | $1 \times 10^{-7}$ M |
| **3** | +33.2 | $2 \times 10^{-6}$ M | $3 \times 10^{-6}$ M |
| **7** | +43.7 | $3 \times 10^{-6}$ M | $3 \times 10^{-8}$ M |
| **8** | +17.8 | $3 \times 10^{-7}$ M | $1 \times 10^{-7}$ M |
| **cisapride** | +40.0 | $3 \times 10^{-8}$ M | $2 \times 10^{-8}$ M |

On the guinea-pig ileum the cumulative dose-response curves to histamine were recorded in the presence of increasing concentrations of the products being examined.

The experimental results, shown in Table 2, highlight how the compounds demonstrate antagonist activity only at very high concentrations, without causing any sensitization of the reactive towards the histamine.

TABLE 2

| Influence on the activity of histamine on guinea-pig ileum | | |
|---|---|---|
| **example** | **effect** | **active concentration** |
| **1** | non competitive antagonism | $1x10^{-4}$ M |
| **2** | mixed antagonism | $1x10^{-4}$ M |
| **3** | non competitive antagonism | $1x10^{-4}$ M |
| **7** | non competitive antagonism | $1x10^{-5}$ M - $1x10^{-4}$ M |
| **8** | mixed antagonism | $1x10^{-5}$ M - $1x10^{-4}$ M |
| **cisapride** | very slight sensitization at low doses | $1x10^{-8}$ M - $1x10^{-7}$ M |

Although they do not demonstrate and cannot predict any prokinetic activity, these last figures make it reasonable to presume that the compounds described have no sedative action involving the central nervous system (mediated by the $H_1$ receptors) to the pharmacologically active concentrations on the physiological peristalsis.

The effect of the products on the contracturing action of the acetylcholine was studied on the isolated ascending colon of rat, taken from Wistar rat sacrificed after 24 hours fasting, washed with De Jalon fluid at 27°C and perfused with the same oxygenated fluid (95% oxygen and 5% carbon dioxide) at the temperature of 27°C. In this case too the cumulative dose-response curves were recorded in the presence of increasing concentrations of the products being studied.

Table 3 below shows how the compounds described in this invention antagonize the acetylcholine at concentrations which are generally higher than the reference cisapride.

TABLE 3

| Influence on the acetylcholine activity on the rat ascending colon | | |
|---|---|---|
| **example** | **effect** | **active concentration** |
| **1** | non competitive antagonism | $1 \times 10^{-4}$ M |
| **2** | non competitive antagonism | $1 \times 10^{-4}$ M |
| **3** | non competitive antagonism | $1 \times 10^{-4}$ M |
| **7** | no effect | ---- |
| **8** | non competitive antagonism | $1 \times 10^{-5}$ - $1 \times 10^{-4}$ M |
| **cisapride** | non competitive antagonism | $1 \times 10^{-6}$ M |

The absence of antagonizing properties towards a mediator involved in physiological peristalsis, at the concentrations which are otherwise active on spontaneous motility (see rabbit jejunum), may further support a prokinetic action of the compounds. The figures also show that the compounds described have no atropine-like effects: this may thus suggest that there are no atropine-like effects *in vivo* involving the central nervous system, at the active concentrations on peristalsis.

The action on the serotonine was evaluated on the fornix of the stomach of rat, also taken from Wistar rat sacrificed after 24 hours fasting, following the technique reported by Vane J.R. in Br. J. Pharmacol. Chemoter. 12, 344, 1957, as previously described.

The results shown in Table 4 below show how the compounds have no effect or act as competitive antagonists but at higher concentrations than the reference cisapride.

TABLE 4

| Influence on the serotonine activity on the fornix of the rat stomach | | |
|---|---|---|
| **example** | **effect** | **active concentration** |
| **1** | no effect | ----- |
| **2** | competitive antagonism | $1x10^{-5}$ M |
| **3** | competitive antagonism | $1x10^{-5}$ M |
| **7** | no effect | ----- |
| **8** | competitive antagonism | $1x10^{-5}$ M - $1x10^{-4}$ M |
| **cisapride** | competitive antagonism | $1x10^{-7}$ M - $1x10^{-6}$ M |

The compounds described interfere with the serotonine only at higher concentrations than those active on the physiological peristalsis and therefore, at these concentrations, they do not interfere in a negative sense on the serotoninergic transmission. The reference cisapride was used in the tests described above, as a control, after being dissolved in tartaric acid at 0.75% at the concentration of 0.15% (w/v).

The prokinetic action was evaluated *in vivo* on Swiss albino mice determining the influence of the products on the intestinal transit of an opaque meal, according to the method described by Macht D.I. and Barba-Gose J. in J. Amer. Pharm. Ass. 20, 558, 1931 and later by Witkin L.B. et al. in J. Pharmacol. Exp. Ther. 133, 400, 1961, modified in that the measure of the tract passed through by the opaque meal was calculated as starting from the cardias rather than from the pylorus.

The method involved the administration of an opaque meal to the mice after a 24 hour fast, through gastric probe, 15 minutes after the administration of the product.

After sacrificing the animals 20 minutes after the administration of the opaque meal, the percentage of the length of the intestine travelled by the opaque meal was calculated. The experimental data, shown in tables 5 and 6, highlight a good variation in percentage with respect to the controls, often higher than the prokinetic activity of cisapride, taken as the reference drug.

TABLE 5

| Test of the progression of the opaque meal in the mouse | | | |
|---|---|---|---|
| **example** | **treatment ($\mu$g/Kg p.o., 15' before the opaque meal)** | **% of intestine (from pylorus to anus) travelled by the opaque meal** | **variation in % with respect to the controls** |
| **1** | 17.3 ($ED_{50}$) | 40.2 | 21.3 |
| **2** | 17.3 | 38.7 | 18.4 |
| **3** | 17.3 | 35.4 | 8.3 |
| **7** | 17.3 ($ED_{50}$) | 49.7 | 28.4* |
| **8** | 17.3 | 41.0 | 0.0 |
| **cisapride** | 19.0 ($ED_{50}$) | 43.6 | 6.0 |

TABLE 6

| Test of the progression of the opaque meal in the mouse evaluated at a dose five times greater than $ED_{50}$ | | |
|---|---|---|
| **Example** | **% of intestine (from pylorus to anus) travelled by the opaque meal** | **Variation % with respect to the controls** |
| **1** | 43.2 | 25.8 |
| **3** | 48.7 | 11.2 |
| **8** | 59.9 | 47.5* |
| **cisapride** | 41.5 | 2.2 |

In particular, the prokinetic activity evaluated at a dose 5 times greater than $ED_{50}$, shown in table 6, demonstrates that compound 8 is considerably more active than the reference drug.

Within the gastro-duodenal tract, the prokinetic activity was evaluated by means of the test of gastric dumping in the rat, according to the method reported by Scarpignato C. in J. Pharmacol 14, 261, 1983. The figures shown in table 7 below for the product of examples 4, 6 and 8 indicate a high level of activity on the gastric dumping in comparison with cisapride.

TABLE 7

| Test of gastric dumping in the rat | | |
|---|---|---|
| **Example** | **Treatment (30' before the administration of the coloured meal)** | **Increase % of the gastric dumping with respect to the controls 20' after the coloured meal** |
| **4** | 12.5 µg/kg<br>25.0 µg/kg<br>50.0 µg/kg<br>75.0 µg/kg | 100.0*<br>115.0*<br>99.8*<br>60.0* |
| **6** | 12.5 µg/kg<br>25.0 µg/kg<br>50.0 µg/kg<br>75.0 µg/kg | 29.7<br>40.5*<br>70.9*<br>35.1 |
| **8** | 5.0 µg/kg<br>10.0 µg/kg<br>50.0 µg/kg<br>100.0 µg/kg<br>200.0 µg/kg | 0.0<br>60.9*<br>74.8*<br>8.7<br>58.7* |
| **cisapride** | 10.0 µg/kg<br>50.0 µg/kg<br>100.0 µg/kg<br>300.0 µg/kg | 23.3<br>1.6<br>23.3<br>39.0* |

Finally the antiemetic activity was evaluated in the pigeon after induction of emesis with cisplatin, according to the method of Preziosi P. et al. in Eur. J. Pharmacol. 221, 343, 1992. The animals were subjected to treatment with the compounds of examples 4, 6 and 8 and with cisapride one hour before treatment with 7.5 mg/kg of cisplatin to induce vomit and the observation was continued for 3 hours after treatment with cisplatin.

The figures shown in table 8 below highlight a good antiemetic activity of the products evaluated.

**TABLE 8**

**Test of vomit from cisplatin in the pigeon**

| Example | Treatment (i.m.) Dose (µg/kg) | N° Anim | Animals that vomit % | Acts of vomit (average/anim.) | Latency of 1st act of vomit | Variation % of the time of latency with respect to the controls |
|---|---|---|---|---|---|---|
| Physiol. | | 16 | 87.5 | 12.3 | 84.5 min. | |
| 4 | 100.0 | 6 | 66.0 | 6.0 | 115 min. | +36.0 |
| 6 | 100.0 | 6 | 83.0 | 7.8 | 127 min.* | +50.5 |
| 8 | 50.0 | 6 | 100.0 | 7.5 | 100 min. | +18.3 |
| | 100.0 | 6 | 100.0 | 7.0 | 100 min. | +18.3 |
| cisapride | 50.0 | 6 | 66.0 | 3.0* | 165.0 min.* | +95.5 |
| | 100.0 | 6 | 66.0 | 2.5* | 150.0 min.* | +77.5 |

The acute toxicity ($LD_{50}$) of the derivatives described in this invention was evaluated according to the method reported by Lietchfield J.T. and Wilcoxon F. in J. Pharmacol. Exp. Ther. 96, 99, 1949, on Sprague-Dawley rats of both sexes, over an observation period of 14 days from the day of treatment.

The results shown in table 9 below demonstrate that the compounds considered have a lower toxicity than cisapride.

TABLE 9

| Study of acute toxicity | |
|---|---|
| **example** | **$LD_{50}$ (mg/Kg/i.v.)** |
| **1** | 50 |
| **2** | 69 |
| **7** | 55 |
| **8** | 60 |
| **cisapride** | 35 |

The biological results obtained in the experiments performed *in vitro* and *in vivo* demonstrate the high prokinetic and antiemetic activity of the derivatives described in this invention in comparison with cisapride, as well as their positive effect on the action of the mediators acetylcholine, serotonine and histamine.

Finally, preliminary studies on the effects as regards behaviour have been performed by means of Irwin's test, according to the method reported by Irwin S. in Gordon Res. Conf. on Medicinal Chem. 1959, 133, 1959, modified in that rats were used instead of mice. After administration by i.p. of 10 mg/Kg of the compounds of examples 4, 6 and 8 no significant variations with respect to the controls were observed in the parameters regarding consciousness, mood, motor activity, excitation of the central nervous system, posture, lack of motor coordination, muscular tone, reflexes and autonomous nervous system.

The examples reported below must be considered as an illustration of this invention and not as a limitation of it.

## EXAMPLE 1

### 5-Chloro-N-[2-(diethylamino)ethyl]-2-methylaminobenzamide citrate

#### a) Synthesis of the 5-chloro-N-methylisatoic anhydride

A solution containing 6 g (30.4 mmoles) of 5-chloroisatoic anhydride in 50 ml of anhydrous dimethylacetamide is added with 1.40 g (56.0 mmoles) of 60% sodium hydride.

The reaction is kept under atmosphere of inert gas at room temperature for 30 minutes and then 2.1 ml (35.0 mmoles) of methyl iodide are added while keeping the reaction at room temperature for further 20 hours.

The solution is poured into 50 ml of an ice-water mixture to precipitate the product which is filtered and washed with ethyl ether.

A product is obtained with a yield of 90%, with the following chemical-physical characteristics:

m.p. = 195 °C

$^1$H-NMR ($CDCl_3$) ppm: 3.59 (3H, s); 7.16 (1H, d); 7.72 (1H, dd); 8.12 (1H, d)

#### b) Synthesis of 5-chloro-N-[2-(diethylamino)ethyl]-2-methylaminobenzamide

5.28 Grammes (25.0 mmoles) of 5-chloro-N-methylisatoic anhydride are suspended in 90 ml of 1,4-dioxane and slowly added, by dripping, with 4.3 ml (30.0 mmoles) of N,N-diethylaminoethylamine; the reaction is kept for 15 minutes at 60 °C.

The raw product , obtained by evaporation of the organic solvent, is purified by chromatography on silica gel with eluent petroleum ether-ethanol-ammonia 85:15:2. After evaporating the elution solvent, a product is obtained with a yield of 85%, with the following chemical-physical characteristics:

$^1$H-NMR ($CDCl_3$) ppm: 1.04 (6H, t); 2.56 (4H, q); 2.62 (2H, t); 2.82 (3H, d); 3.40 (2H, m); 6.56 (1H, d); 6.82 (1H, broad); 7.23 (1H, dd); 7.29 (1H, d); 7.44 (1H, broad)

IR (film) $cm^{-1}$: 3340, 2960, 2800, 1640, 1570, 1510, 1400, 1270, 1250, 1170, 1070, 880, 810, 750

**c) Synthesis of 5-chloro-N-[2-(diethylamino)ethyl]-2-methylaminobenzamide citrate**

3.50 Grammes (12.0 mmoles) of 5-chloro-N-[2-(diethylamino)ethyl]-2-methylaminobenzamide dissolved in 5 ml of ethyl alcohol are joined, at the temperature of 4 °C, by 15 ml of a solution containing 2.85 g (13.5 mmoles) of monohydrate citric acid in a mixture of ethyl ether-ethyl alcohol in the ratio 5:1.

The product crystallizes immediately and is recovered through filtration with a yield of 93%.

It shows the following chemical-physical characteristics:

m.p. = 160 °C

$^1$H-NMR (DMSO) ppm: 1.15 (6H, t); 2.55 (4H, m); 2.75 (3H, d); 3.07 (6H, m); 3.50 (2H, m); 6.65 (1H, d); 7.32 (1H, dd); 7.58 (1H, d); 7.68 (1H, broad); 8.63 (1H, broad)

$^{13}$C-NMR (DMSO) ppm: 8.9 (**$CH_3$**); 29.4 (N-**$CH_3$**); 34.6 (N-**$CH_2$**-$CH_2$); 44.1 (N-**$CH_2$**-$CH_3$); 45.7 (CO-**$CH_2$**-$CH_2$); 49.9 (N-$CH_2$-**$CH_2$**); 71.6 (**C**-OH); 112.7 (**CH arom.**); 115.5 (**C arom.**); 117.7 (**C arom.**); 127.8 (**CH arom.**); 132.5 (**CH arom.**); 149.3 (**C arom.**); 168.8 (N-**C=O**); 171.9 (O-**C=O**); 177.2 (O-C-**C(O)=O**)

IR (nujol) $cm^{-1}$: 3350, 1730, 1640, 1580, 1510, 1460, 1380, 1220, 1170, 800, 720

| Elementary analysis: | | | | | |
|---|---|---|---|---|---|
| calculated | C 50.51 | H 6.36 | N 8.84 | O 26.93 | Cl 7.36 |
| found | C 50.54 | H 6.32 | N 8.92 | O 26.89 | Cl 7.29 |

**EXAMPLE 2**

**5-Chloro-N-[3-(diethylamino)propyl]-2-methylaminobenzamide citrate**

**a) Synthesis of 5-chloro-N-[3-(diethylamino)propyl]-2-methylamino benzamide**

1.50 Grammes (7.1 mmoles) of 5-chloro-N-methylisatoic anhydride are suspended in 20 ml of 1,4-dioxane and added with 1.34 ml (8.4 mmoles) of 3-diethylamino-1-propylamine.

The reaction is kept for 5 hours at 60 °C; the raw product is recovered by evaporating the solvent.

The solid is dissolved in ethyl acetate, washed with an aqueous solution at pH 11, recovered through evaporation of the organic solvent and purified through silica gel chromatography with eluent petroleum ether-isopropanol-ammonia 85:15:1. After evaporating the elution solvent a product is obtained, with a yield of 81%, with the following chemical-physical characteristics:

$^1$H-NMR ($CD_3COCD_3$) ppm: 1.03 (6H, t); 1.72 (2H, m); 2.53 (4H, q); 2.58 (2H, t); 2.82 (3H, d); 3.40 (2H, m); 6.65 (1H, d); 7.23 (1H, dd); 7.48 (1H, d); 7.90 (1H, broad); 8.40 (1H, broad)

**b) Synthesis of 5-chloro-N-[3-(diethylamino)propyl]-2-methylamino benzamide citrate**

1.46 Grammes (4.9 mmoles) of 5-chloro-N-[3-(diethylamino)propyl]-2-methylaminobenzamide dissolved in 5 ml of ethyl alcohol are added, at the temperature of 4 °C, with 10 ml of a solution containing 1.24 grammes (5.9 mmoles) of monohydrate citric acid in a mixture of ethyl ether-ethyl alcohol in the ratio 5:1.

The product crystallizes immediately and is recovered by filtration with a yield of 89%.

It shows the following chemical-physical characteristics:

m.p. = 125 °C

$^1$H-NMR (DMSO) ppm: 1.18 (6H, t); 1.87 (2H, m); 2.56 (4H, s); 2.75 (3H, s); 3.07 (6H, m); 3.30 (2H, q); 5.50 (3H, broad); 6.62 (1H, d); 7.31 (1H, dd); 7.60 (1H, d); 8.52 (1H, broad).

$^{13}$C-NMR (DMSO) ppm: 8.9 (**$CH_3$**); 23.8 (**$CH_2$**); 29.6 (**N-$CH_3$**); 36.6 (N-**$CH_2$**-$CH_2$); 44.5 (CO-**$CH_2$**-C); 46.5 (N-**$CH_2$**-$CH_3$); 49.1 (N-$CH_2$-**$CH_2$**); 71.8 (**C**-OH); 112.6 (**CH arom.**); 116.2 (**C arom.**); 117.7 (**C arom.**); 127.8 (**CH arom.**); 132.2 (**CH arom**); 149.1 (**C arom.**); 168.4 (N-**C=O**); 171.8 (O-**C=O**); 177.2 (O-C-**C(O)=O**).

IR (nujol) $cm^{-1}$: 2980, 1730, 1640, 1580, 1520, 1410, 1330, 1270, 1100, 1030, 870, 800

## EXAMPLE 3

### 5-Chloro-2-methylamino-N-[2-(1-pyrrolidinyl)ethyl]benzamide citrate

#### a) Synthesis of 5-chloro-2-methylamino-N-[2-(1-pyrrolidinyl)ethyl] benzamide

2.00 Grammes (9.5 mmoles) of 5-chloro-N-methylisatoic anhydride are suspended in 25 ml of dioxane and, at room temperature, are added by dripping with 1.43 ml (11.3 mmoles) of N-(2-aminoethyl)pyrrolidine and then the reaction is kept at room temperature for 6 hours.

The raw solid is recovered through evaporation of the solvent, redissolved in methylene chloride and washed with an aqueous solution of sodium hydroxide at pH 9.

The solid, obtained through evaporation of the solvent, is purified through chromatography on a silica gel column by using petroleum ether-isopropanolammonia 170:30:1 as eluent.

After evaporation of the elution solvent a substance is obtained, with a yield of 80%, with the following chemical-physical characteristics:

m.p. = 111 °C

$^1$H-NMR ($CD_3COCD_3$) ppm: 1.70 (4H, m); 2.55 (6H, m); 2.82 (3H, d); 3.45 (2H, m); 6.64 (1H, d); 7.25 (1H, dd); 7.50 (1H, d); 7.70 (1H, broad)

#### b) Synthesis of 5-chloro-2-methylamino-N-[2-(1-pyrrolidinyl)ethyl] benzamide citrate

1.97 Grammes (7.0 mmoles) of 5-chloro-2-methylamin-N-[2-(1-pyrrolidinyl)ethyl]benzamide dissolved in 15 ml of ethyl alcohol are added, at the temperature of 4 °C, with 15 ml of a solution containing 1.62 g (7.7 mmoles) of monohydrate citric acid in a mixture of ethyl ether-ethyl alcohol in the ratio 5:1.

The product crystallizes immediately and is recovered through filtration with a yield of 87%.

It shows the following chemical-physical characteristics:

m.p. = 160 °C

$^1$H-NMR (DMSO) ppm: 1.95 (4H, m); 2.55 (4H, m); 2.80 (3H, s); 3.26 (6H, t); 3.50 (2H, m); 6.65 (1H, d); 7.30 (1H, dd); 7.62 (1H, d); 8.70 (1H, broad)

$^{13}$C-NMR (DMSO) ppm: 22.9 (**$CH_2$**); 29.6 (N-**$CH_3$**); 36.0 (N-$CH_2$-**$CH_2$**); 44.4 (CO-**$CH_2$**-C-); 53.7 (N-**$CH_2$**-$CH_2$); 72.0 (**C**-OH); 112.7 (**CH arom.**); 116.0 (**C arom.**); 117.7 (**C arom.**); 128.0 (**CH arom.**); 132.5 (**CH arom.**); 149.2 (**C arom.**); 168.7 (N-**C=O**); 172.0 (O-**C=O**); 177.3 (O-C-**C(O)=O**)

IR (nujol) $cm^{-1}$: 3400, 1725, 1630, 1580, 1530, 1400, 1225, 1090, 1060, 1000, 790

## EXAMPLE 4

### 5-Chloro-N-[2-(diethylamino)ethyl]-2-[N'-methyl)benzylamino]benzamide citrate

#### a) Synthesis of 5-chloro-N-[2-(diethylamino)ethyl]-2-[N'-methyl) benzyl amino]benzamide

2.00 Grammes (7.2 mmoles) of 5-chloro-N-[2-(diethylamino)ethyl]-2-methylaminobenzamide are suspended in 20 ml of a mixture water-ethanol in the ratio 1:1 containing 480 mg (12.0 mmoles) of sodium hydroxide and 1.20 g (8.8 mmoles) of potassium carbonate.

After 30 minutes of reflux, 1.04 ml (8.8 mmoles) of benzyl bromide are slowly dripped.

After 20 hours of reflux, the product is recovered through evaporation of the solvent and purified through silica gel chromatography with eluent petroleum ether-isopropanol-ammonia in the ratio 85:15:1. After evaporating the elution solvent, an oily substance is obtained, with a yield of 27%, with the following chemical-physical characteristics:

$^1$H-NMR ($CD_3COCD_3$) ppm: 1.02 (6H, t); 2.55 (4H, q); 2.63 (2H, t); 2.70 (3H, s); 3.52 (2H, m); 4.22 (2H, s); 6.70 (1H, s); 7.15-7.40 (7H, m); 7.95 (1H, d); 9.60 (1H, broad)

#### b) Synthesis of 5-chloro-N-[2-(diethylamino)ethyl]-2-[N'-methyl) benzyl amino]benzamide citrate

480 Milligrammes (1.28 mmoles) of 5-chloro-N-[2-(diethylamino)ethyl]-2-[N'-methyl)benzylamino]-benzamide in 5 ml of ethyl alcohol are added, at the temperature of 4 °C, with 10 ml of a solution containing 300 mg (1.44 mmoles) of monohydrate citric acid in a mixture of ethyl ether-ethyl alcohol in the ratio 5:1.

The product, which crystallizes immediately, is recovered through filtration with a yield of 79%.

It shows the following chemical-physical characteristics:
m.p. = 155 °C
$^1$H-NMR (DMSO) ppm: 1.30 (6H, t); 2.78 (3H, s); 3.15-3.45 (6H, t+q); 3.82 (2H, t); 4.45 (2H, s); 6.70 (1H, d); 7.32 (1H, dd); 7.53 (1H, d)
$^{13}$C-NMR (DMSO) ppm: 9.3 (**$CH_3$**); 37.8 (N-**$CH_2$**-$CH_2$); 41.4 (N-**$CH_3$**); 44.3 (CO-**$CH_2$**-C-); 46.9 (N-**$CH_2$**-$CH_3$); 50.1 (N-$CH_2$-**$CH_2$**); 59.7 (N-**$CH_2$**-Ph); 72.0 (**C**-OH); 122.2 (**CH arom.**); 125.7 (**C arom.**); 127.4 (**CH arom.**); 128.5 (**CH arom.**); 129.3 (**CH arom.**); 130.0 (**C arom.**); 130.7 (**CH arom.**); 137.6 (**C arom.**); 149.2 (**C arom.**); 167.1 (N-**C=O**); 171.8 (O-**C=O**); 177.2 (O-C-**C(O)=O**)
IR (nujol) cm$^{-1}$: 1730, 1650, 1590, 1410, 1270, 1030, 820, 800, 700

## EXAMPLE 5

### 5-Chloro-N-[2-(diethylamino)ethyl]-2-[N'-methyl)cyclohexylmethylamino] benzamide citrate

#### a) Synthesis of 5-Chloro-N-[2-(diethylamino)ethyl]-2-[N'-methyl)cyclohexyl methylamino]benzamide

2.00 Grammes (7.0 mmoles) of 5-chloro-N-[2-(diethylamino)ethyl]-2-methylaminobenzamide dissolved in 20 ml of anhydrous dimethylacetamide are added with 374 mg (15.5 mmoles) of sodium hydride and, after 30 minutes, 1.40 ml (15.2 mmoles) of bromomethylcyclohexane are dripped into the solution.

After 48 hours at room temperature water and ice are added and the raw product is extracted with methylene chloride.

The product recovered through evaporation of the solvent is purified through silica gel chromatography with petroleum ether-isopropanol-ammonia in the ratio 95:5:1 as eluent. After evaporating the elution solvent, an oily substance is obtained, with a yield of 30%, with the following chemical-physical characteristics:
$M^+$ = 377

#### b) Synthesis of 5-chloro-N-[2-(diethylamino)ethyl]-2-[N'-methyl)cyclohexyl methylamino]benzamide citrate

300 Milligrams (0.8 mmoles) of 5-chloro-N-[2-(diethylamino)ethyl]-2-[N'-methyl)cyclohexylmethylamino]-benzamide dissolved in 4 ml of 1:1 mixture of ethanol and chloroform are added, at the temperature of 4 °C, with 10 ml of a mixture of ethyl ether-ethanol in the ratio 5:1 containing 189 mg (0.9 mmoles) of monohydrate citric acid.

The product which crystallizes immediately is recovered through filtration with a yield of 85% and shows the following chemical-physical characteristics:
m.p. : 180 °C
$^1$H-NMR (DMSO) ppm: 1.12 (11H, t+m); 1.65 (5H, m); 2.75-3.28 (10H, m); 2.78 (3H, s); 3.68 (3H, m); 4.37 (2H, s); 6.85 (1H, d); 7.34 (1H, dd); 7.75 (1H, d); 8.92 (1H, broad)

## EXAMPLE 6

### 5-Chloro-2-methylamino-N-[2-(4-morpholinyl)ethyl]benzamide citrate

#### a) Synthesis of 5-chloro-2-methylamino-N-[2-(4-morpholinyl)ethyl] benzamide

2.00 Grammes (9.5 mmoles) of 5-chloromethylisatoic anhydride suspended in 15 ml of dioxane are added, by dripping, with 1.4 ml (15.8 mmoles) of N-2(aminoethyl)morpholine. After two hours at room temperature the solid is recovered through silica gel chromatography with eluent n-hexane-isopropanolammonia in the ratio 85:15:1 in gradient till the ratio 60:40:1.

After evaporating the elution solvent the product is obtained with a yield of 76% and shows the following chemical-physical characteristics:
m.p. : 135 °C
$^1$H-NMR ($CD_3COCD_3$) ppm: 2.32-2.68 (6H, m); 2.82 (3H, d); 3.11 (1H, broad); 3.27-3.70 (6H, m); 6.64 (1H, d); 7.26 (1H, dd); 7.49 (1H, d); 7.63 (1H, broad)

**b) Synthesis of 5-chloro-2-methylamino-N-[2-(4-morpholinyl)ethyl] benzamide citrate**

1.00 Gramme (3.4 mmoles) of 5-chloro-2-methylamino-N-[2-(4-morpholinyl)ethyl]benzamide dissolved in 5 ml of a 1:1 mixture of ethanol and chloroform is added, at the temperature of 4°C, with 5 ml of a mixture of ethyl ether and ethanol in the ratio 5:1 containing 776 mg (3.7 mmoles) of monohydrate citric acid.

The product which crystallizes immediately is recovered through filtration with a yield of 89% and shows the following chemical-physical characteristics:

m.p. : 192°C

$^1$H-NMR (DMSO) ppm: 2.40-2.98 (10H, m); 2.79 (3H, s); 3.44 (2H, s); 3.69 (4H, s); 6.63 (1H, d); 7.31 (1H, d); 7.60 (1H, s); 8. 15 (1H, broad); 8.50 (1H, broad)

$^{13}$C-NMR (DMSO) ppm: 29.6 (**$CH_3$**); 35.5 (N-**$CH_2$**-$CH_2$); 43.6 (CO-**$CH_2$**-C-); 52.7 (O-$CH_2$-**$CH_2$**); 56.7 (N-$CH_2$-**$CH_2$**); 65.3 (O-**$CH_2$**-$CH_2$); 72.4 (**C**-OH); 112.6 (**CH arom.**); 116.2 (**C arom.**); 117.7 (**C arom.**); 127.8 (**CH arom.**); 132.2 (**CH arom.**); 149.1 (**C arom.**); 168.3 (N-**C=O**); 171.7 (O-**C=O**); 175.8 (O-C-**C(O)=O**)

## EXAMPLE 7

### 5-Chloro-N-[2-(diethylamino)ethyl]-2-methylaminobenzamide oxalate

3.40 Grammes (12.0 mmoles) of 5-chloro-N-[2-(diethylamino)ethyl]-2-methylaminobenzamide in 5 ml of ethyl alcohol are added, at the temperature of 4°C, with a solution containing 1.67 g (13.2. mmoles) of dihydrate oxalic acid in 15 ml of ethyl ether-ethyl alcohol in the ratio 5:1.

The product, which crystallizes immediately, is recovered through filtration. A product is obtained, with a yield of 70%, with the following chemical-physical characteristics:

$M^+$ = 284 (loss of oxalate ion)

m.p. = 140°C

$^1$H-NMR (DMSO) ppm: 1.20 (6H, t); 2.75 (3H, s); 3.00-3.30 (6H, t+q); 3.55 (2H, m); 6.65 (1H, d); 7.32 (1H, dd); 7.62 (1H, d); 7.50-8.30 (3H, broad); 8.82 (1H, broad)

$^{13}$C-NMR (DMSO) ppm: 8.4 (**$CH_3$**); 29.4 (**$CH_3$**); 34.0 (N-**$CH_2$**-$CH_2$); 46.6 (N-**$CH_2$**-$CH_3$); 49.1 (N-$CH_2$-**$CH_2$**); 112.7 (**CH arom.**); 115.4 (**C arom.**); 117.8 (**C arom.**); 128.0 (**CH arom.**); 132.5 (**CH arom.**); 149.3 (**C arom.**); 165.4 (N-**C=O**); 168.7 (O-**C=O**)

IR (nujol) $cm^{-1}$: 3320, 1640, 1580, 1520, 1470, 1380, 1270, 1250, 880, 810, 710

| Elementary analysis: | | | | | |
|---|---|---|---|---|---|
| calculated | C 51.46 | H 6.48 | N 11.26 | O 21.43 | Cl 9.37 |
| found | C 50.52 | H 6.43 | N 11.68 | O 21.36 | Cl 9.30 |

## EXAMPLE 8

### 5-Chloro-N-[3-(diethylamino)propyl]-2-methylaminobenzamide oxalate

2.00 Grammes (6.7 mmoles) of 5-chloro-N-[3-(diethylamino)propyl]-2-methylaminobenzamide in 7 ml of ethyl alcohol are added, at the temperature of 4°C, with a solution containing 1.02 g (8.0 mmoles) of dihydrate oxalic acid in 15 ml of ethyl ether-ethyl alcohol in the ratio 5:1.

The product, which crystallizes immediately, is recovered through filtration. A product is obtained, with a yield of 90%, with the following chemical-physical characteristics:

$M^+$ = 297 (loss of oxalate ion)

m.p. = 151°C

$^1$H-NMR (DMSO) ppm: 1.15 (6H, t); 1.86 (2H, m); 2.75 (3H, s); 3.03 (6H, m); 3.25 (2H, m); 6.62 (1H, d); 7.30 (1H, dd); 7.60 (1H, d); 8.60 (1H, broad)

$^{13}$C-NMR (DMSO) ppm: 8.4 (**$CH_3$**); 23.3 ($CH_2$-**$CH_2$**-$CH_2$); 29.3 (NH-**$CH_2$**-$CH_2$); 36.5 (N-**$CH_3$**); 46.1 (N-**$CH_2$**-$CH_3$); 48.6 (N-$CH_2$-**$CH_2$**); 112.6 (**CH arom.**); 116.2 (**C arom.**); 117.8 (**C arom.**); 127.9 (**CH arom.**); 132.2 (**CH arom.**); 149.2 (**C arom.**); 165.4 (N-**C=O**); 168.5 (O-**C=O**)

IR (nujol) $cm^{-1}$: 3400, 3310, 1710, 1630, 1510, 1450, 1370, 1240, 700

| Elementary analysis: | | | | | |
|---|---|---|---|---|---|
| calculated<br>found | C 52.69<br>C 52.02 | H 6.77<br>H 6.67 | N 10.85<br>N 10.53 | O 20.66<br>O 20.40 | Cl 9.03<br>Cl 8.95 |

## Claims

1. Compounds with benzamidic structure endowed with prokinetic and antiemetic activity of general formula

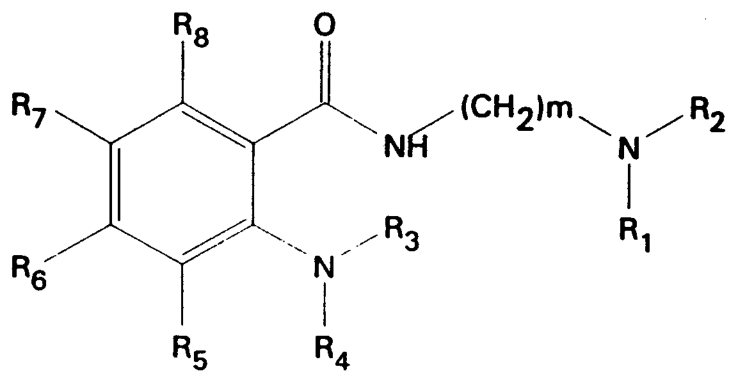

I

and their pharmacologically acceptable salts in which *m* represents a whole number between 1 and 4, $R_1$ and $R_2$, independently, represent an atom of hydrogen or a ($C_1$-$C_6$)-alkyl radical, with a lineare or ramified chain, or $R_1$ and $R_2$ taken together with the atom of nitrogen form a heterocyclic ring, $R_3$ and $R_4$, independently, represent an atom of hydrogen or a ($C_1$-$C_{10}$)-alkyl radical, with linear, ramified or cyclic chain, or a benzyl radical and $R_5$, $R_6$, $R_7$ and $R_8$, independently, represent an atom of hydrogen or a ($C_1$-$C_6$)-alkyl radical, with linear or ramified chain, or an atom of halogen.

2. Compounds according to claim 1 in which $R_1$ and $R_2$, independently, represent a ($C_1$-$C_3$)-alkyl radical or an atom of hydrogen or taken together with the atom of nitrogen form a heterocyclic ring, $R_5$, $R_6$, and $R_8$ represent an atom of hydrogen, $R_7$ represents an atom of halogen and *m* is a whole number selected between 2 and 3.

3. Compounds according to claim 2 characterized by the fact that the halogen is chlorine.

4. Compounds according to claim 3 which are 5-chloro-N-[2-(diethylamino)ethyl]-2-methylaminobenzamide and the oxalate and citrate salts thereof.

5. Compounds according to claim 3 which are 5-chloro-N-[3-(diethylamino)propyl]-2-methylaminobenzamide and the oxalate and citrate salts thereof.

6. Compounds according to claim 3 which are 5-chloro-2-methylamino-N-[2-(1-pyrrolidinyl)ethyl]-benzamide and the citrate salts thereof.

7. Compounds according to claim 3 which are 5-chloro-N-[2-(diethylamino)ethyl]-2-[N'-methyl)-benzylamino]benzamide and the citrate salt thereof.

8. Compounds according to claim 3 which are 5-chloro-N-[2-(diethylamino)ethyl]-2-[(N'-methyl)-cyclohexylmethylamino]benzamide and the citrate salt thereof.

9. Compounds according to claim 3 which are 5-chloro-2-methylamino-N-[2-(4-morpholinyl)ethyl]-benzamide and the citrate salt thereof.

European Patent Office

# PARTIAL EUROPEAN SEARCH REPORT

which under Rule 45 of the European Patent Convention shall be considered, for the purposes of subsequent proceedings, as the European search report

Application Number: EP 94 10 5463

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| X | US-A-3 170 955 (R. K. RICHARDS)<br>* column 1, line 27 - line 27; claim 4; examples III,VI *<br>--- | 1-3 | C07C237/34<br>C07D295/13<br>A61K31/165<br>A61K31/40<br>A61K31/535 |
| X | CHEMICAL ABSTRACTS, vol. 112, no. 13, 26 March 1990, Columbus, Ohio, US; abstract no. 118466r,<br>T. ADACHI ET AL.<br>page 701 ;<br>* abstract *<br>& JP-A-1 168 655 (TEIKOKU CHEMICAL INDUSTRY)<br>--- | 1-3 | |
| X | FR-A-1 431 815 (CENTRE NATIONAL DE LA RECHERCHE SCIENTIFIQUE)<br>* claim 1 *<br>--- | 1 | |
| X | FR-A-1 458 953 (AZIENDE CHIMICHE RIUNITE ANGELINI FRANCESCO)<br>* claims 1-6,9,12 *<br>--- | 1-3 | |
| X | FR-A-1 485 847 (MILES LABORATORIES)<br>* see example, e.g. 4a, 14a, 19a, 28a *<br>---<br>-/-- | 1-3 | |

TECHNICAL FIELDS SEARCHED (Int.Cl.5)

C07C
C07D

## INCOMPLETE SEARCH

The Search Division considers that the present European patent application does not comply with the provisions of the European Patent Convention to such an extent that it is not possible to carry out a meaningful search into the state of the art on the basis of some of the claims

Claims searched completely :

Claims searched incompletely :

Claims not searched :

Reason for the limitation of the search:

see sheet C

| Place of search | Date of completion of the search | Examiner |
|---|---|---|
| THE HAGUE | 30 June 1994 | Seufert, G |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

European Patent Office

# PARTIAL EUROPEAN SEARCH REPORT

Application Number
EP 94 10 5463

| DOCUMENTS CONSIDERED TO BE RELEVANT | | | CLASSIFICATION OF THE APPLICATION (Int.Cl.5) |
|---|---|---|---|
| Category | Citation of document with indication, where appropriate, of relevant passages | Relevant to claim | |
| X | GB-A-460 250 (I. G. FARBENINDUSTRIE)<br>* examples *<br>--- | 1 | |
| X | JOURNAL OF ORGANIC CHEMISTRY<br>vol. 26 , 1961 , EASTON US<br>pages 613 - 615<br>S. M. GADEKAR, E. ROSS 'Some Halogenated 1,2,3-Benzotriazin-4(3H)ones'<br>* page 614, table I, example 2,3,5,6 *<br>--- | 1-3 | |
| X | JOURNAL OF ORGANIC CHEMISTRY<br>vol. 29 , 1964 , EASTON US<br>pages 2717 - 2720<br>A. A. SANTILLI, T. S. OSDENE '1H-2,1,3-Benzothiadiazin-4(3H)-one 2-Oxides'<br>* table I, example i *<br>--- | 1-3,9 | TECHNICAL FIELDS SEARCHED (Int.Cl.5) |
| X | JOURNAL OF MEDICINAL CHEMISTRY<br>vol. 12 , 1969 , WASHINGTON US<br>pages 936 - 938<br>S. HAYAO ET AL. 'Hypotensive, Antiadrenergic, and Antihistaminic 3-Substituted 2-Methyl-(or 2-Phenyl-) 4(3H)-quinazolones'<br>* page 936, compound IV; page 938, left column, line 11 - line 18 *<br>--- | 1-3 | |
| X | CHEMICAL ABSTRACTS, vol. 79, no. 11, 17 September 1973, Columbus, Ohio, US;<br>abstract no. 66290g,<br>N. HIROSE ET AL.<br>page 444 ;<br>* abstract and RN 50886-42-5, 49666-29-7, 30646-50-5 *<br>& CHEM. PHARM. BULL.<br>vol. 21, no. 5 , 1973<br>pages 1005 - 13<br>--- | 1-3 | |

-/--

European Patent Office

# PARTIAL EUROPEAN SEARCH REPORT

Application Number
EP 94 10 5463

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| A | CHEMICAL AND PHARMACEUTICAL BULLETIN. vol. 40, no. 1 , 1992 , TOKYO JP pages 202 - 211 J. SAKAGUCHI ET AL 'Synthesis, Gastrointestinal Prokinetik Activity and Structure-Activity Relationships of Novel N-[[2-(Dialkylamino)ethoxy]benzyl]benzamide Derivatives' * the whole document; see especially page 202, line 1 - line 22, example II-30, page 205, last lines * --- | 1-9 | |
| A | EP-A-0 105 599 (ADRIA LABORATORIES) * page 1, line 15 - page 2, line 14 * * page 3, line 1 - line 5 * --- | 1-9 | TECHNICAL FIELDS SEARCHED (Int.Cl.5) |
| A | US-A-4 093 734 (G. KRÜGER) * column 13, line 67 - column 14, line 6; claims * * examples, e.g. 58 * ----- | 1-9 | |

EPO FORM 1503 03.82 (P04C10)

EP 94 10 5463
-C-

INCOMPLETE SEARCH

Claims searched completely: 2-9

Claim searched incompletely: 1

The large number of theoretically conceivable compounds resulting from the combination of the claimed substituents $R^5$ - $R^8$ precludes a comprehensive search.
For economic reasons the search has been limited to compounds with $R^7$ = Cl, $R^{5,6,8}$ = H
(Rule 45 EPC, Guidelines Exam. Part B, Chapt. III, 3.6, 3.7)